(19) Europäisches Patentamt
European Patent Office
Office européen des brevets

(11) EP 1 763 301 B3

(12) # NEUE EUROPÄISCHE PATENTSCHRIFT

Nach dem Beschränkungsverfahren (B3-1)

(45) Hinweis auf die Patenterteilung:
**02.11.2011 Patentblatt 2011/44**

(45) Veröffentlichungstag und Bekanntmachung des Hinweises auf die Entscheidung über den Antrag auf Beschränkung:
**B3-1 29.05.2013 Patentblatt 2013/22**

(21) Anmeldenummer: **05758033.4**

(22) Anmeldetag: **16.06.2005**

(51) Int Cl.:
***A01N 25/34*** (2006.01) ***A01N 47/22*** (2006.01)
***A01N 53/00*** (2006.01) ***A01N 51/00*** (2006.01)
*A01N 53/00* (2006.01) *A01N 51/00* (2006.01)
*A01N 47/22* (2006.01) *A01N 25/34* (2006.01)
*A01N 51/00* (2006.01) *A01N 25/34* (2006.01)
*A01N 47/22* (2006.01) *A01N 25/34* (2006.01)

(86) Internationale Anmeldenummer:
**PCT/EP2005/006465**

(87) Internationale Veröffentlichungsnummer:
**WO 2006/000335 (05.01.2006 Gazette 2006/01)**

(54) **WIRKSTOFFHALTIGE FESTE FORMKÖRPER ZUR ÄUßERLICHEN ANWENDUNG GEGEN PARASITEN AN TIEREN**

ACTIVE SUBSTANCE-CONTAINING SOLID SHAPED BODIES FOR EXTERNAL USE AGAINST PARASITES IN ANIMALS

CORPS FAÇONNES SOLIDES CONTENANT DES PRINCIPES ACTIFS, A USAGE EXTERNE, SERVANT A LUTTER CONTRE LES PARASITES CHEZ LES ANIMAUX

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**HR**

(30) Priorität: **29.06.2004 DE 102004031325**

(43) Veröffentlichungstag der Anmeldung:
**21.03.2007 Patentblatt 2007/12**

(73) Patentinhaber: **Bayer Animal Health GmbH**
**51368 Leverkusen (DE)**

(72) Erfinder:
- **SIRINYAN, Kirkor**
**51467 Bergisch Gladbach (DE)**
- **LÖHR, Reinhold**
**51469 Bergisch Gladbach (DE)**

(74) Vertreter: **Bröcher, Dirk Joachim**
**Gille Hrabal**
**Patentanwälte**
**Brucknerstrasse 20**
**40593 Düsseldorf (DE)**

(56) Entgegenhaltungen:
**EP-A- 0 537 998** **WO-A-02/49436**
**DE-A1- 2 519 126** **FR-A- 2 746 585**
**US-A- 4 536 388** **US-A- 6 001 382**

Bemerkungen:
Die Akte enthält technische Angaben, die nach dem Eingang der Anmeldung eingereicht wurden und die nicht in dieser Patentschrift enthalten sind.

EP 1 763 301 B3

## Beschreibung

Die vorliegende Erfindung betrifft wirkstoffhaltige Formkörper zur äußerlichen Anwendung gegen Parasiten an Tieren.

Wirkstoffhaltige Formkörper zur Bekämpfung von Parasiten bei Tieren sind seit langem bekannt. Derartige Formkörper werden beispielsweise in WO 2002/78443, EP-A 539295, EP-A 763325, EP-A 1289363, EP-A 211207, EP-A 124404, EP-A 979605, FR 2729833, US 5620696, EP-A 576267, EP-A 569791, EP-A 542078, EP-A 470467, EP-A 251472, EP-A 50782, US 5858387 und EP-A 542080 beschrieben. Der Nachteil der bislang beschriebenen Verfahren sowie Abmischungen zur Herstellung der wirkstoffhaltigen Formkörper liegt darin, dass sie bei Verwendung der konventionellen Polyolefin- oder Vinylharz-Matrices den zusätzlichen Einsatz von Phthalsäureestem, wie Dimethylphthalat und Dioctylphthalat (siehe beispielsweise WO 01/787065, EP-A-0 211 207 und EP-A-0 569 791) erfordern. Die hierzu eingesetzten, aus technologischer Sicht brauchbaren Phthalate sind bekannterweise toxikologisch nicht ganz unbedenklich. Sie können bei nicht sachgerechter Handhabung zu Risiken bei der Herstellung der Formkörper und bei deren Anwendung führen, darüber hinaus besteht die Gefahr der Umweltkontamination.

Es ist daher wünschenswert, die besagten Phtalate durch weniger toxische, umweltverträgliche Einsatzstoffe zu ersetzen. Vorzugsweise sollen diese neuen Einsatzstoffe die Migration der Wirkstoffe aus der Polymermatrix erhöhen und somit die Wirksamkeit (Residual- und Knock-down-Wirkung) der Formkörper verbessern, ohne die guten physikalisch-chemischen Eigenschaften der Polyolefinmatrix negativ zu beeinflussen.

Überraschenderweise wurde nun gefunden, dass dieses Ziel mittels bestimmter Fettsäureester mehrwertiger Alkohole (z.B. Di- und Triglyceride oder Propylenglykol-Ester) erreicht werden kann.

Aufgrund ihres physikalisch-chemischen Aufbaues sind solche Fettsäureester polare Verbindungen während die genannten Polyolefine und Vinylharze relativ unpolare Kunststoffe sind. Der Fachmann erwartet, dass polare Verbindungen und unpolare Kunststoffe unverträglich sind. Solche Kombinationen führen in vielen Fällen zu Entmischung/Phasentrennung, was wiederum zur erheblichen Verschlechterung der physikalisch-chemischen Eigenschaften der Polymermatrix, wie E-Modul, Duktilität, Zug-Dehnung, Entnehmbarkeit aus der jeweiligen Formmasse, sowie zu spontaner Wirkstoff-Migration aus der Kunststoffmatrix und somit zu erheblicher Verschlechterung der Langzeitwirksamkeit führt. Es war daher überraschend, dass der Einsatz der genannten, polaren Fettsäureester mehrwertiger Alkohole weder die physikalisch-chemischen Eigenschaften noch die Langzeitwirksamkeit der Formkörper negativ beeinflusst. In vielen Fällen wurde sogar, entgegen der Lehrmeinung, eine Verbesserung in Verarbeitbarkeit und Langzeitwirkung beobachtet.

Die Erfindung betrifft:

Feste Formkörper zur äußerlichen Anwendung gegen Parasiten an Tieren auf Basis einer Polyolefin-Matrix, enthaltend

- ein Capryl-/Caprinsäuretriglycerid und/oder Propylenglykoldicaprylat/-dicaprat,
- einen oder mehrere Wirkstoffe
- sowie ggf. weitere Hilfs- und Zusatzstoffe.

Diese Glycerin- bzw. Propylenglykolester der Capryl-/Caprinsäure weisen einen Viskositätsbereich (20°C) von 5 bis 40, bevorzugt 8 bis 35, besonders bevorzugt 9 bis 13 mPa.s auf. Diese Ester sind unter der Handelsbezeichnung Miglyol 840 (Propylenglykoloctanoatdecanoat, CAS-Nr. 68583-51-7) und Miglyol 812 (Capryl-Caprinsäure-Triglyceride, CAS-Nr. 73398-61-5) der Fa. Sasol Germany GmbH/Witten erhältlich.

Die erfindungsgemäßen Formkörper enhalten die Fettsäureester in Anteilen von 1 bis 25 Gew.%, bevorzugt 5 bis 17,5 Gew.-%, besonders bevorzugt 5 bis 12,5 Gew.-% (bezogen auf die jeweilige Gesamtabmischungmasse).

Bei den erfindungsgemäßen festen Formkörpern handelt es sich z.B. um Halsbänder, Anhänger für Halsbänder (Medaillons), Ohrmarken, Bänder zur Befestigung an Gliedmaßen oder Körperteilen, Klebestreifen und -folien oder Abziehfolien. Besonders bevorzugt sind Medaillons und insbesondere Halsbänder.

Als Trägersubstanz oder Basis für die Formkörper kommen thermoplastische und flexible thermoplastische Polyolefine und Elastomere in Frage. Als solche seien genannt Polyvinylharze, EPDM (Ethylen-/Propylen-Dien-Terpolymer), Polyethylen (z.B. HDPE oder LLDPE), Polypropylen, die mit den oben genannten Wirkstoffen ausreichend verträglich sind.

Die Polymeren müssen eine ausreichende Festigkeit und Biegsamkeit haben, um beim Formen nicht zu reißen oder brüchig zu werden. Sie müssen von ausreichender Haltbarkeit sein, um gegen normale Abnutzung beständig zu sein. Außerdem müssen die Polymere eine ausreichende Migration des Wirkstoffs an die Oberfläche des Formkörpers zulassen.

Zu den Polyvinylharzen gehören Polyvinylhalogenide, wie Polyvinylchlorid, Polyvinylchlorid-Vinylacetat und

Polyvinylfluorid; Polyvinylbenzole, wie Polystyrol und Polyvinyltoluol.

Weitere als Matrix der erfindungsgemäßen Formkörper geeignete Kunststoffe sind thermoplastische Elastomere. Dies sind Werkstoffe, die elastomere Phasen in thermoplastisch verarbeitbaren Polymeren entweder physikalisch eingemischt oder chemisch eingebunden enthalten. Man unterscheidet Polymerblends, in denen die elastomeren Phasen Bestandteil des polymeren Gerüsts sind. Durch den Aufbau der thermoplastischen Elastomere liegen harte und weiche Bereiche nebeneinander vor. Die harten Bereiche bilden dabei eine kristalline Netzstruktur oder eine kontinuierliche Phase deren Zwischenräume von elastomeren Segmenten ausgefüllt sind. Aufgrund dieses Aufbaus haben diese Werkstoffe kautschukähnliche Eigenschaften. In diesem Zusammenhang sei auf Thermoplastische Polyolefine (TPO) und auf Styrol-Block Copolymere (s. beispielsweise EP 542078) hingewiesen.

Erfindungsgemäß bevorzugt sind Polyvinylchlorid, Polypropylen, Polyethylen und EPDM; ganz besonders bevorzugt ist Polyvinylchlorid.

Für die Herstellung der Formkörper auf der Basis von Polyolefinen, insbesondere von Polyvinylharzen können in besonderen Fällen zusätzlich übliche Weichmacher, die bekannterweise zum Weichmachen von festen Vinylharzen verwendetwerden, eingesetztwerden. Derzu verwendende Weichmacher hängt von dem Harz und seiner Verträglichkeit mit dem Weichmacher ab. Geeignete zusätzliche Weichmacher sind beispielsweise Phosphorsäureester, Adipinsäureester, wie z.B. Diiso- und n-Butyladipat. Es können auch andere Ester, wie die Ester von Azelainsäure, Maleinsäure, Ricinolsäure, Myristinsäure, Palmitinsäure, Ölsäure, Sebacinsäure, Stearinsäure und Trimellithsäure, sowie komplexe lineare Polyester, polymere Weichmacher und epoxydierte Sojabohnenöle verwendet werden.

Zusätzliche Weichmacher werden ggf. in Mengen von etwa 5 bis 50 Gew.-%, vorzugsweise etwa 15 bis 45 Gew.-% der gesamten Zusammensetzung eingesetzt.

In den Formkörper können noch weitere übliche Bestandteile, wie Stabilisierungsmittel, Schmiermittel, Formtrennmittel, Füllstoffe und Färbematerialien, enthalten sein; in der Regel werden dadurch die grundlegenden Eigenschaften der Zusammensetzung nicht wesentlich verändert.

Geeignete Stabilisierungsmittel sind Antioxidationsmittel und Mittel, welche die Bänder vor ultravioletter Strahlung und unerwünschtem Abbau während der Bearbeitung, wie Strangpressen schützen. Einige Stabilisierungsmittel, wie epoxidierte Sojabohnenöle, dienen außerdem als sekundäre Weichmacher.

Als Schmiermittel können beispielsweise Stearate, Stearinsäure und Polyethylene mit niedrigem Molekulargewicht verwendet werden. Diese Bestandteile werden üblicherweise in einer Konzentration bis zu etwa 5 Gew.-% der gesamten Zusammensetzung verwendet.

Bei der Herstellung der Formkörper werden vorzugsweise die verschiedenen Bestandteile nach bekannten Verfahren gemischt und nach bekannten Strangpress- oder Spritzgussverfahren formgepresst.

Die Wahl des Verarbeitungsverfahrens zur Herstellung der Formkörper richtet sich technisch grundsätzlich nach den rheologischen Eigenschaften des polymeren Trägermaterials und der Form des gewünschten Formkörpers. Die Verarbeitungsverfahren können nach der Verarbeitungstechnologie oder nach der Art der Formgebung eingestellt werden. Bei der Verfahrenstechnologie kann man die Verfahren nach den bei ihnen durchlaufenden rheologischen Zuständen unterteilen. Danach kommen für viskose polymere Trägermaterialien Gießen, Pressen, Spritzgießen und Auftragen und für elastoviskose Polymere Spritzgießen, Strangpressen (Extrudieren), Kalandrieren, Walzen und gegebenenfalls Kneten in Frage. Nach Art der Formgebung eingeteilt, lassen sich die erfindungsgemäßen Formkörper durch Gießen, Tauchen, Pressen, Spritzgießen, Extrudieren, Kalandrieren, Prägen, Biegen, Tiefziehen etc. herstellen. Ein Beschichten von festen Grundträgern kommt ebenfalls in Frage.

Diese Verarbeitungsverfahren sind an sich bekannt und bedürfen keiner näheren Erklärung. Im Prinzip gelten für andere Polymere die Erläuterungen, die oben beispielhaft für Polyvinylharze gemacht wurden.

Die erfindungsgemäßen festen Formkörper eignen sich bei günstiger Warmblütertoxizität zur Bekämpfung von Parasiten, die in der Tierhaltung und Tierzucht bei Haus- und Nutztieren sowie Zoo-, Labor-, Versuchs- und Hobbytieren vorkommen. Sie sind gegen normal sensible und resistente Arten sowie gegen alle oder einzelne Entwicklungsstadien der genannten Tiere wirksam.

Parasiten sind insbesondere Arthropoden. Bevorzugt werden die erfindungsgemäßen festen Formulierungen eingesetzt zur Bekämpfung von Ektoparasiten.

Zu den oben erwähnten Ektoparasiten gehören: Schildzecken, Lederzecken, Räudemilben, Laufmilben, Fliegen (stechend und leckend), parasitierende Fliegenlarven, Läuse, Haarlinge, Federlinge, Zecken und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp.,

Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattella germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Ixodes Holocyclus, Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die Formkörper eignen sich generell für die Bekämpfung von Parasiten bei den meisten Tierarten, bevorzugt bei Warmblütern, insbesondere Säugetieren. Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere; Pelztiere wie z. B. Nerze, Chinchilla, Waschbär; Geflügel wie z.B. Hühner, Gänse, Puten, Enten.

Zu Labor- und Versuchstieren gehören Rinder, Mäuse, Ratten, Meerschweinchen, Goldhamster, Hunde und Katzen.

Zu den Hobbytieren gehören Pferde, Hunde, Katzen, Mäuse, Ratten, Meerschweinchen, Goldhamster, Hasen und Kaninchen.

Die erfindungsgemäßen Mittel eignen sich insbesondere zur Behandlung von Rind, Hund und Katze, und zwar vorzugsweise zur Bekämpfung von Zecken und/oder Flöhen.

Die Anwendung kann sowohl prophylaktisch als auch therapeutisch erfolgen.

Die Formkörper enthalten üblicherweise den Wirkstoff in Konzentrationen von jeweils 0,1 bis 30 Gew.-%, bevorzugt von 1 bis 20 Gew.-%, besonders bevorzugt von 2 bis 15,0 Gew.-%, bezogen auf die Gesamtmasse der festen Formulierung.

Die Formkörper enthalten üblicherweise Wirkstoffkombinationen in Gesamtkonzentrationen von 1 bis 35 Gew.-%, bevorzugt 2 bis 25 Gew.-%, besonders bevorzugt von 2,5 bis 17,5 Gew.-% bezogen auf die Gesamtmasse der festen Formulierung.

Die neuen festen Formkörper können Wirkstoffe, wie Insektizide, Akarizide, Lockstoffe, Sterilantien, Bakterizide, Nematizide, Fungizide etc. enthalten. Zu den Insektiziden, Akaradiziden sowie Wachstumsinhibitoren zählen beispielsweise Phosphorsäureester, Carbamate, Carbonsäureester, synthetische oder natürliche Pyrethroide, Neonicotinoide (auch als Chlornicotinyle bezeichnet; dazu gehören z.B. Chlorpyridin-, Chlorthiazol- undTetrahydrofuran-Verbindungen), Pyroximate, Phenylether, Phenylharnstoffe, durch Mikroorganismen hergestellte Stoffe u.a..

Günstige Wirkstoffe und Cowirkstoffe sind z.B. die folgenden:

Insektizide / Akarizide / Nematizide /Wachstuminhinitoren:

Abamectin, Acephate, Acetamiprid, Acrinathrin, Alanycarb, Aldicarb, Aldoxycarb, Alphacypermethrin, Alphamethrin, Amitraz, Avermectin, AZ 60541, Azadirachtin, Azamethiphos, Azinphos A, Azinphos M, Azocyclotin,

Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,

Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,

Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusatsodium, Dofenapyn,

Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,

Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenvalerate, Fipronil, Fluazinam, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,

Granuloseviren

Halofenozide, HCH, Heptenophos, Hexaflumuron, Hexythiazox, Hydroprene,

Imidacloprid, Isazofos, Isofenphos, Isoxathion, Ivermectin,

Kernpolyederviren

Lambdacyhalothrin, Lufenuron

Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos, Moxidectin,

Naled, Nitenpyram, Nithiazine, Novaluron

Omethoat, Oxamyl, Oxydemethon M

Paecilomycesfumosoroseus, ParathionA, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,

Quinalphos,

Ribavirin

Salithion, Sebufos, Selamectin, Silafluofen, Spinosad, Sulfotep, Sulprofos,

Taufluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Ti 435, Tralocythrin, Tralomethrin, Triarathene, Triazamate, Triazophos, Triazuron, Trichlophenidine, Trichlorfon, Triflumuron, Trimethacarb,

Vamidothion, Vaniliprole, Verticillium lecanii

YI 5302

YRC 2894

Zetacypermethrin, Zolaprofos

(1 R-cis)-[5-(Phenylmethyl)-3-furanyl]-methyl-3-[(dihydro-2-oxo-3(2H)-furanyliden)-methyl]-2,2-dimethylcyclopro-pancarboxylat

(3-Phenoxyphenyl)-methyl-2,2,3,3-tetramethylcyclopropanecarboxylat

1-[(2-Chlor-5-thiazolyl)methyl]tetrahydro-3,5-dimethyl-N-nitro-1,3,5-triazin-2(1H)-imin

2-(2-Ch lor-6-fluorphenyl)-4-[4-(1,1-dimethylethyl)phenyl]-4,5-dihydro-oxazol

2-(Acetlyoxy)-3-dodecyl-1,4-naphthalindion

2-Chlor-N-[[[4-(1-phenylethoxy)-phenyl]-amino]-carbonyl]-benzamid

2-Chlor-N-[[[4-(2,2-dichlor-1,1-difluorethoxy)-phenyl]-amino]-carbonyl]-benzamid

3-Methylphenyl-propylcarbamat

4-[4-(4-Ethoxyphenyl)-4-methylpentyl]-1-fluor-2-phenoxy-benzol

4-Chlor-2-(1,1-dimethylethyl)-5-[[2-(2,6-dimethyl-4-phenoxyphenoxy)ethyl]thio]-3(2H)-pyridazinon

4-Chlor-2-(2-chlor-2-methylpropyl)-5-[(6-iod-3-pyridinyl)methoxy]-3(2H)-pyridazinon 4-Chlor-5-[(6-chlor-3-pyridinyl)methoxy]-2-(3,4-dichlorphenyl)-3(2H)-pyridazinon

Bacillus thuringiensis strain EG-2348

Benzoesäure [2-benzoyl-1-(1,1-dimethylethyl)-hydrazid

Butansäure 2,2-dimethyl-3-(2,4-dichlorphenyl)-2-oxo-1-oxaspiro[4.5]dec-3-en-4-yl-ester

[3-[(6-Chlor-3-pyridinyl)methyl]-2-thiazolidinyliden]-cyanamid

Dihydro-2-(nitromethylen)-2H-1,3-thiazine-3(4H)-carboxaldehyd

Ethyl-[2-[[1,6-dihydro-6-oxo-1-(phenylmethyl)-4-pyridazinyl]oxy]ethyl]-carbamat

N-(3,4,4-Trifluor-1-oxo-3-butenyl)-glycin

N-(4-Chlorphenyl)-3-[4-(difluormethoxy)phenyl]-4,5-dihydro-4-phenyl-1H-pyrazol-1-carboxamid

N-[(2-Chlor-5-thiazolyl)methyl]-N'-methyl-N"-nitroguanidin

N-Methyl-N'-(1-methyl-2-propenyl)-1,2-hydrazindicarbothioamid

N-Methyl-N'-2-propenyl-1,2-hydrazindicarbothioamid

O,O-Diethyl-[2-(dipropylamino)-2-oxoethyl]-ethylphosphoramidothioat

Bevorzugte Pyrethroide sind Cyfluthrin, β-Cyfluthrin, Flumethrin.

Bevorzugte Neonicotinoide (Chloronicotinyle) sind:

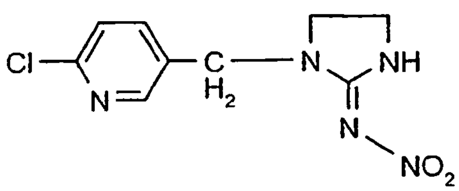

Imidacloprid

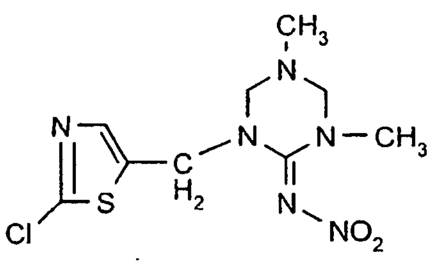

AKD 1022

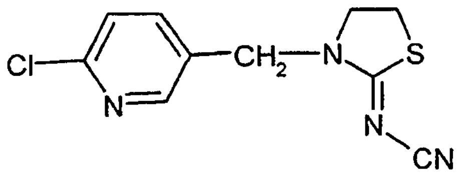

Thiacloprid

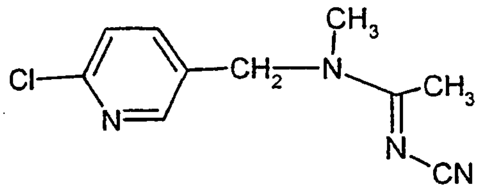

Acetamiprid

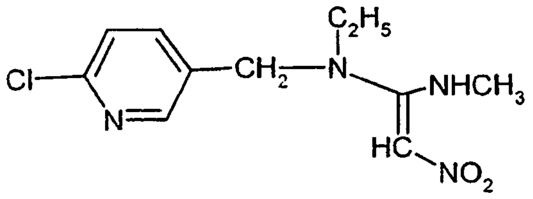

Nitenpyram (Ti 304)

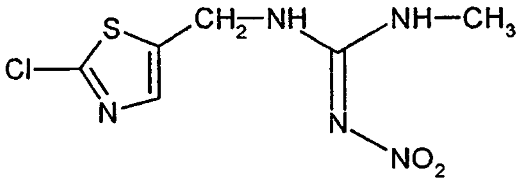

Clothianidin (Ti 435)

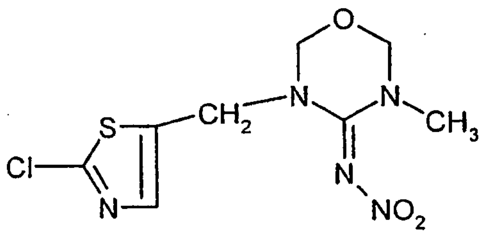

Thiamethoxam

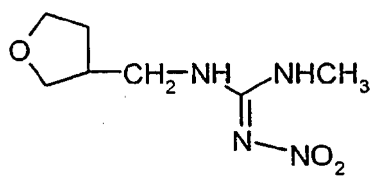

Dinotefuran

Imidacloprid, Thiacloprid, Acetamiprid und Nitenpyram sind Vertreter der Chlorpyridin-Neonicotinoide; Thiametoxam, Clothianidin und AKD 1022 sind Vertreter der Chlorthiazol-Neonicotinoide und Dinotefuran ist ein Vertreter der Tetrahydrofuran-Neonicotinoide.

Als weiteres bevorzugtes Insektizid sei Propoxur genannt.

Bevorzugte Wachstumsinhibitoren sind Pyriproxyfen, Methopren und Triflumuron, sie eignen sich insbesondere für die Anwendung in Kombination mit einem anderen Insektizid/Akarizid.

Bevorzugter Synergist ist Piperonylbutoxid; auch die Synergisten werden selbstverständlich in Kombination mit entsprechenden Wirkstoffen eingesetzt.

Die Verwendung der genannten Cowirkstoffe und Synergisten mit den genannten Wirkstoffen ist prinzipiell bekannt, siehe z. B. WO 00/02453, WO 95/33380,WO 95/07615, EP-A 569791, EP-A 0 736 252, EP-A 470467 und EP-A 251472.

Als weitere Wirkstoffe seien Pyrazoloxime und Benzoylharnstoffe genannt.

Geeignete Pyrazoloxime mit insektizider und akarizider Wirkung werden z.B. in der EP-A-0 234 045 beschrieben.

Zu den Benzoylhamstoffen gehören Verbindungen der Formel (I):

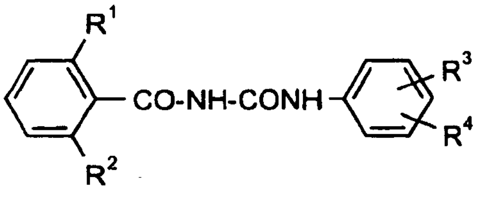

(-I)

wobei

$R^1$ für Halogen steht,

$R^2$ für Wasserstoff oder Halogen steht,

$R^3$ für Wasserstoff, Halogen oder $C_{1-4}$-Alkyl steht,

$R^4$ für Halogen, 1-5-Halogen-$C_{1-4}$-alkyl, $C_{1-4}$-Alkoxy, 1-5-Halogen-$C_{1-4}$-alkoxy, $C_{1-4}$-Alkylthio, 1-5-Halogen-$C_{1-4}$-alkylthio, Phenoxy oder Pyridyloxy die gegebenenfalls substituiert sein können durch Halogen, $C_{1-4}$-Alkyl, 1-5-Halogen-$C_{1-4}$-alkyl, $C_{1-4}$-Alkoxy, 1-5-Halogen-$C_{1-4}$-alkoxy, $C_{1-4}$-Alkylthio, 1-5-Halogen-$C_1$-$C_4$-alkylthio.

Die genannten Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemate lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Die erfindungsgemäßen Verbindungen können gegebenenfalls sowohl als cis- als auch als trans-Isomere vorliegen. Auch wenn nur eines der Isomere dargestellt ist, sind erfindungsgemäß immer das cis- und das trans-Isomere gemeint.

Die erfindungsgemäßen Formkörper eignen sich hervorragend zur Durchführung von äußerlichen bzw. dermalen Behandlungen an Tieren, insbesondere Hunden, Katzen und Rindern. Sie weisen üblicherweise eine Dicke von 0,25 - 3,5 mm, vorzugsweise 0,75 - 2,5 mm auf. Sie zeichnen sich aus durch ihre ganz hervorragende Lagerungsstabilität von 3-5 Jahren in allen Klimazonen. Sie zeichnen sich weiterhin durch ihre einfache Applizierbarkeit, sehr gute biologische Langzeitwirksamkeit von üblicherweise bis zu neun Monaten sowie ihre gute Umwelt- insbesondere Gewässer- und Warmblüterverträglichkeit aus.

## Beispiele

Beispiel **1**

Zusammensetzung:

| | |
|---|---|
| 2-Isopropoxyphenyl-N-methylcarbamat (Propoxur) | 10 g |
| Di-n-butyladipat | 21 g |
| Propylenglycoloctanoatdecanoat (Handelsbezeichnung: Miglyol 840, Fa. Sasol/Witten) | 9 g |
| Epoxidiertes Sojabohnenöl | 2 g |
| Stearinsäure | 1 g |
| PVC | 56 g |
| Pigmentmischung | 1 g |

Herstellung:

Die Mischung aus 2-Isopropoxyphenyl-N-methylcarbamat, Pigmentmischung und PVC wird in einem Mischer mit dem Gemisch aus Di-n-butyladipat, Propylenglycoloctanoatdecanoat und epoxidiertem Sojabohnenöl gemischt. Man mischt unter Wärme solange, bis die Mischung homogen ist. Das Erwärmen fördert das Einziehen der Weichmachermischung in das PVC. Nach der anschließenden homogenen Verteilung der Stearinsäure wird die Mischung im Spritzguss zu Halsbändern geformt.

**Beispiel 2**

Zusammensetzung:

| | |
|---|---|
| 2-Isopropoxyphenyl-N-methylcarbamat | 10 g |
| Flumethrin | 2,5 g |
| Di-n-butyladipat | 21 g |
| Propylenglycoloctanoatdecanoat | 9 g |
| Epoxidiertes Sojabohnenöl | 2 g |
| Stearinsäure | 1 g |
| PVC | 54 g |
| Pigmentmischung | 0,5 g |

Herstellung:

Die Mischung aus 2-Isopropoxyphenyl-N-methylcarbamat, Pigmentmischung und PVC wird in einem Mischer mit dem Gemisch aus Di-n-butyladipat, Propylenglycoloctanoatdecanoat, epoxidiertem Sojabohnenöl und Flumethrin gemischt. Man mischt unter Wärme solange, bis die Mischung homogen ist. Das Erwärmen fördert das Einziehen der Wirkstoff-Weichmachermischung in das PVC. Nach der anschließenden homogenen Verteilung der Stearinsäure wird die Mischung im Spritzguss zu Halsbändern geformt. Gleiche Mischung wird im Extruder zu Endlosbändern oder Platten extrudiert, die vom Hersteller oder Anwender auf eine der Applikationsform gemäße Länge konfektioniert wird. Aus dem Extrudat werden Formkörper geschnitten oder gestanzt, die in einer Medaillonform eng an das Tier gehängt wird.

**Beispiel 3**

Zusammensetzung:

| | |
|---|---|
| Flumethrin | 2,5 g |
| Di-n-butyladipat | 21 g |
| Propylenglycoloctanoatdecanoat | 9 g |
| Epoxidiertes Sojabohnenöl | 2 g |
| Stearinsäure | 1 g |
| PVC | 64 g |
| Pigmentmischung | 0,5 g |

Herstellung:

Die Mischung aus Pigmentmischung und PVC wird in einem Mischer mit dem Gemisch aus Di-n-butyladipat, Propylenglycoloctanoatdecanoat, epoxidiertem Sojabohnenöl und Flumethrin gemischt. Man mischt unter Wärme solange, bis die Mischung homogen ist. Das Erwärmen fördert das Einziehen der Wirkstoff-Weichmachermischung in das PVC. Nach der anschließenden homogenen Verteilung der Stearinsäure wird die Mischung zu Endlosbändern und Platten extrudiert, die vom Hersteller oder Anwender auf eine der Applikationsform gemäße Länge konfektioniert wird. Aus dem Extrudat werden Formkörper geschnitten oder gestanzt, die in einer Medaillon- bzw. Ohrmarkenform eng an das Tier gehängt wird.

**Beispiel 4**

Zusammensetzung:

| | |
|---|---|
| Imidacloprid | 10 g |
| Flumethrin | 5 g |
| Di-n-butyladipat | 21 g |
| Propylenglycoloctanoatdecanoat | 9 g |
| Epoxidiertes Sojabohnenöl | 2 g |
| Stearinsäure | 1 g |
| PVC | 51 g |
| Pigmentmischung | 1 g |

Herstellung:

Die Mischung aus Imidacloprid, Pigmentmischung und PVC wird in einem Mischer mit dem Gemisch aus Di-n-butyladipat, Propylenglycoloctanoatdecanoat, epoxidiertem Sojabohnenöl und Flumethrin gemischt. Man mischt unter Wärme solange, bis die Mischung homogen ist. Das Erwärmen fördert das Einziehen der Wirkstoff-Weichmachermischung in das PVC. Nach der anschließenden homogenen Verteilung der Stearinsäure wird die Mischung im Spritzguss zu Halsbändern und Medallions geformt.

Bei den in den Beispielen genannten Pigmentmischungen handelt es sich in den

Beispielen 1 und 4 um eine Mischung aus handelsüblichen Eisenoxiden, in den

Beispielen 2 und 3 um ein Gemisch aus handelsüblichem Titandioxid und Eisenoxid.

**Wirksamkeitsversuche**

Zur Durchführung von Wirksamkeitsversuchen gegen Flöhe und Zecken wurden Hunde mit Halsbändern bzw. Rinder mit einem ihrer Körpergröße angepassten Extrudat gemäß den Beispielen 1, 2, 3, 4, behandelt. Die Behandlung erfolgte über die Befestigung eines Formkörpers in Form eines Halsbandes (ca. 1,4cm breiter Streifen) am Hals der Tiere. Die Streifen wurden möglichst eng (mit einem fingerbreit Zwischenraum) an den Hals der Tiere angepasst. Medaillons wurden perforiert und an einem herkömmlichen, Wirkstoff-freien Halsband befestigt. Das Medaillon wurde so plaziert, dass es im vorderen Halsbereich Kontakt mit dem Fell des Tieres hatte.

**Beispiel A: Wirksamkeit gegen Flöhe *(Ctenocephalides felis*) am Hund**

An den Tagen -4 und -1 werden Hunde mit ca. 100 adulten, nüchternen *Ctenocephalides felis* pro Hund infestiert. Dabei werden die Flöhe auf den Nacken des Tieres ausgebracht.

Am Tag 0 wird der Infestationserfolg am Hund überprüft, indem am wachen Tier nach Flöhen gesucht wird. Die Zahl der lebenden Flöhe wird protokolliert.

Nach der Zählung der Flöhe werden die Tiere behandelt. Die Hunde der Kontrollgruppe werden nicht behandelt. Die zu prüfenden Arzneimittel werden den Tieren als Halsband oder als Medaillon verabreicht. Halsbänder und Medaillon verbleiben am Tier bis zum Versuchsende am Tag 170. Es wird pro Tier jeweils nur 1 Halsband oder 1 Medaillon appliziert. Es werden nur klinisch gesunde Tiere verwendet.

Am Tag 1 und Tag 2 werden alle Hunde auf lebende Flöhe und Zecken überprüft. Die Ergebnisse werden in den Rohdaten festgehalten.

An den Tagen 14, 28, 56, 84, 112, 140 und 168 werden alle Hunde mit ca. 100 adulten, nüchternen *Ctenocephalides felis* pro Hund reinfestiert. Jeweils einen und zwei Tage nach Reinfestation werden alle Hunde auf lebende Flöhe kontrolliert. Die Ergebnisse werden in den Rohdaten protokolliert.

Für die Berechnung der Wirksamkeit wird eine modifizierte Formel nach Abbott benutzt:

$$\text{Wirksamkeit \%} = \frac{\text{Ø Anzahl Flöhe KG} - \text{Ø Anzahl Flöhe BG}}{\text{Ø Anzahl Flöhe KG}} \text{ X } 100$$

KG: Kontrollgruppe
BG: Behandlungsgruppe

Mit den gemäß der in den Formulierungsbeispielen 1, 2, 3, 4 hergestellten, als Halsband und als Medaillon applizierten, wirkstoffhaltigen Formkörpern können Wirksamkeiten >90% gegen *Ctenocephalides felis* über 5-6 Monate

erzielt werden.

**Beispiel B: Wirksamkeit gegen Zecken *(Ixodes ricinus)* am Hund**

AmTag-1 werden Hunde mit 2% Rompun® (Bayer AG) (0,1 ml/kg Körpergewicht) sediert. Nachdem alle Hunde sediert sind (nach ca. 10-15 Minuten) werden sie in Transportboxen überführt und *50 Ixodes ricinus* (25 ♀, 25♂) pro Hund auf den Nacken des Tieres ausgebracht. Die Tiere werden nach ca. 1 ½ Stunden wieder aus der Transportkiste in den Käfig gesetzt.

Am Tag 0 wird der Infestationserfolg am Hund überprüft, indem am wachen Tier nach Zecken gesucht wird. Intensiv wird dabei gesucht im Kopf- und Ohrenbereich inkl. Ohrenfalte, im Bereich des Nackens, am Unterbauch, an der Unterbrust, an der seitlichen Flanke sowie zwischen den Zehen und an den Gliedmaßen. Die Zahl der angesogenen lebenden Zecken wird protokolliert. Tote Zecken werden entfernt.

Nach der Zählung der Zecken werden die Tiere behandelt. Die Hunde der Kontrollgruppe werden nicht behandelt. Die zu prüfenden Arzneimittel werden den Tieren als Halsband oder als Medaillon verabreicht. Halsbänder und Medaillon verbleiben am Tier bis zum Versuchsende am Tag 170. Es wird pro Tier jeweils nur 1 Halsband oder 1 Medaillon appliziert. Es werden nur klinisch gesunde Tiere verwendet.

Am Tag 1 und Tag 2 werden alle Hunde auf lebende und tote angesogende Zecken überprüft. Die Ergebnisse werden in den Rohdaten festgehalten. Am Tag 2 werden alle lebenden und toten Zecken vom Hund entfernt.

An den Tagen 14, 28, 56, 84, 112, 140 und 168 werden alle Hunde mit jeweils 50 *Ixodes ricinus* (25♀, 25♂) pro Hund reinfestiert. Jeweils einen und zwei Tage nach Reinfestation werden alle Hunde auf lebende und tote angesogene Zecken kontrolliert. Die Ergebnisse werden in den Rohdaten protokolliert. Am 2.ten Tag nach Reinfestation werden alle lebenden und toten Zecken vom Hund entfernt.

Für die Berechnung der Wirksamkeit wird eine modifizierte Formel nach Abbott benutzt:

$$\text{Wirksamkeit \%} = \frac{\text{Ø Anzahl Zecken KG} - \text{Ø Anzahl Zecken BG}}{\text{Ø Anzahl Zecken KG}} \text{ X } 100$$

KG: Kontrollgruppe
BG: Behandlungsgruppe

Mit den gemäß der in den Formulierungsbeispielen 1, 2, 3, 4 hergestellten, als Halsband und als Medaillon applizierten, wirkstoffhaltigen Formkörpern können Wirksamkeiten >90% gegen *Ixodes ricinus* über 5-6 Monate erzielt werden.

**Beispiel C: Wirksamkeit gegen den australischen Zecken *(Ixodes holocyclus)* am Rind**

Am Tag -1 werden Hunde mit 2% Rompun® (BayerAG) (0,1 ml/kg Körpergewicht) sediert. Nachdem alle Rinder sediertsind (nach ca. 10-15 Minuten) und 10 *Ixodes holocyclus* (5♀, 5♂) pro Rind auf den Nacken des Tieres ausgebracht.

Am Tag 0 wird der Infestationserfolg am Rind überprüft, indem am wachen Tier nach Zecken gesucht wird. Intensiv wird dabei gesucht im Kopf- und Ohrenbereich inkl. Ohrenfalte, im Bereich des Nackens, am Unterbauch, an der Unterbrust, an der seitlichen Flanke sowie zwischen den Zehen und an den Gliedmaßen. Die Zahl der angesogenen lebenden Zecken wird protokolliert. Tote Zecken werden entfernt.

Nach der Zählung der Zecken werden die Tiere behandelt. Die Rinder der Kontrollgruppe werden nicht behandelt. Die zu prüfenden Arzneimittel werden den Tieren als Halsband verabreicht. Halsbänder verbleiben am Tier bis zum Versuchsende am Tag 170. Es wird pro Tier jeweils nur 1 Halsband appliziert. Es werden nur klinisch gesunde Tiere verwendet.

Am Tag 1 und Tag 2 werden alle Hunde auf lebende und tote angesogende Zecken überprüft. Die Ergebnisse werden in den Rohdaten festgehalten. Am Tag 2 werden alle lebenden und toten Zecken vom Hund entfernt.

An den Tagen 14, 28, 56, 84, 112, 140 und 168 werden alle Rinder mit jeweils 50 *Ixodes holocyclus* (5♀, 5♂) pro Rind reinfestiert. Jeweils einen und zwei Tage nach Reinfestation werden alle Hunde auf lebende und tote angesogene Zecken kontrolliert. Die Ergebnisse werden in den Rohdaten protokolliert. Am 2.ten Tag nach Reinfestation werden alle lebenden und toten Zecken vom Rind entfernt.

Für die Berechnung der Wirksamkeit wird eine modifizierte Formel nach Abbott benutzt:

$$\text{Wirksamkeit \%} = \frac{\text{Ø Anzahl Zecken KG} - \text{Ø Anzahl Zecken BG}}{\text{Ø Anzahl Zecken KG}} \times 100$$

KG: Kontrollgruppe
BG: Behandlungsgruppe

Mit den gemäß der in den Formulierungsbeispielen 1, 2, 3, 4 hergestellten, als Halsband applizierten, wirkstoffhaltigen Formkörpern können Wirksamkeiten >90% gegen *Ixodes holocyclus* über 5-6 Monate erzielt werden.

**Patentansprüche**

**1.** Feste Formkörper zur äußerlichen Anwendung gegen Parasiten an Tieren auf Basis einer Polyolefin-Matrix, enthaltend

- ein Capryl-/Caprinsduretriglycerid und/oder Propylenglykoldicaprylat/-dicaprat.
- einen oder mehrere Wirkstoffe
- sowie ggf. weitere Hilfs- und Zusatzstoffe.

**2.** Feste Formkörper gemäß Anspruch 1 auf Basis einer Polyvinylharz-Matrix.

**3.** Feste Formkörper gemäß Anspruch 1 auf Basis einer Polyvinylchlorid-Matrix, bestehend aus

- 9 Gew.-% Propylenglycoloctanoatdecanoat,
- 10 Gew.-% Imidacloprid und 5 Gew.-% Flumethrin,
- 21 Gew.-% Di-n-butyladipat, 2 Gew.-% epoxidiertes Sojabohnenöl und 1 Gew.-% Stearinsäure,
- 1 Gew.-% Eisenoxid, sowie
- 51 Gew.-% der Polyvinylchlorid-Matrix.

**4.** Feste Formkörper gemäß Anspruch 1 zur Verwendung zur dermalen Bekämpfung von Parasiten an Tieren.

**Claims**

**1.** Solid moulded bodies which are for external use against parasites on animals, which are based on a polyolefin matrix and which comprise

- a caprylic/capric acid triglyceride and/or propylene glycol dicaprylate/dicaprate
- one or more active compounds
- and, where appropriate, additional auxiliary substances and additives,

**2.** Solid moulded bodies according to Claim 1 which are based on a polyvinyl resin matrix.

**3.** Solid moulded bodies according to Claim 1 which are based on a polyvinyl chloride matrix consisting of

- 9 wt.-% propylene glycol octanoate decanoate,
- 10 wt.-% Imidacloprid,
- 21 wt.-% di-n-butyl adipate, 2 wt.-% epoxidized soybean oil and 1 wt.-% stearic acid,
- 1 wt.-% iron oxide, and
- 51 wt.-% of the polyvinyl chloride matrix.

**4.** Solid moulded bodies according to Claim 1 for use for dermally controlling parasites on animals.

## Revendications

1. Corps façonnés solides à usage externe contre les parasites chez les animaux, à base d'une matrice de polyoléfine, contentant

    - un triglycéride d'acide caprylique/caprique et/ou un dicaprylate/dicaprate d'éthylène glycol,
    - un ou plusieurs principes actifs,
    - ainsi qu'éventuellement d'autres substance auxiliaires et d'addition.

2. Corps façonnés solides selon la revendication 1, à base d'une matrice en résine polyvinylique.

3. Corps façonnés solides selon la revendication 1, à base d'une matrice en chlorure de polyvinyle consistant en

    - 9 % en poids d'octanoate décanoate de propylène glycol,
    - 10 % en poids d'Imidoclopride et 5 % en poids de fluméthrine,
    - 21 % en poids de l'adipate de di-n-butyle, 2 % en poids de l'huile de soja époxydée et 1 % en poids de l'acide stéarique,
    - 1 % en poids de l'oxyde de fer et
    - 51 % en poids de la matrice en chlorure de polyvinyle,

4. Corps façonnés solides selon la revendication à utiliser pour la lutte dermique contre les parasites chez les animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 200278443 A
- EP 539295 A
- EP 763325 A
- EP 1289363 A
- EP 211207 A
- EP 124404 A
- EP 979605 A
- FR 2729833
- US 5620696 A
- EP 576267 A
- EP 569791 A
- EP 542078 A
- EP 470467 A
- EP 251472 A
- EP 50782 A
- US 5858387 A
- EP 542080 A
- WO 01787065 A
- EP 0211207 A
- EP 0569791 A
- WO 0002453 A
- WO 9533380 A
- WO 9507615 A
- EP 0736252 A
- EP 0234045 A